# EUROPEAN PATENT SPECIFICATION

(11) **EP 4 338 692 B1**
(45) Date of publication and mention of the grant of the patent: **10.12.2025**
(21) Application number: 23195483.5
(22) Date of filing: 05.09.2023
(51) Int. Cl.: A61B 17/221, A61B 18/14

(54) **RETRIEVAL OF MATERIAL FROM VESSEL LUMENS**
ENTNAHME VON MATERIAL AUS GEFÄSSLUMEN
EXTRACTION DE MATÉRIAU DES LUMIÈRES VASCULAIRES

(30) Priority: 14.09.2022 US 202217932234
(43) Date of publication of application: 20.03.2024
(73) Proprietor: Covidien LP, Mansfield, MA 02048 (US)
(72) Inventor: Skillrud, Eric, Irvine, CA 92617 (US); Kashyap, Varun Umesh, Irvine, CA 92617 (US)
(74) Representative: Gray, James

(56) References cited:
- WO-A1-2021/118868
- WO-A1-2022/058873
- US-A1- 2010 234 842
- US-A1- 2016 199 127
- US-A1- 2020 390 457

## Description

### TECHNICAL FIELD

The present technology relates generally to devices and methods for removing obstructions from body lumens. Some embodiments of the present technology relate to devices and methods for removal of clot material from blood vessels.

### BACKGROUND

Many medical procedures use medical device(s) to remove an obstruction (such as clot material) from a body lumen, vessel, or other organ. An inherent risk in such procedures is that mobilizing or otherwise disturbing the obstruction can potentially create further harm if the obstruction or a fragment thereof dislodges from the retrieval device. If all or a portion of the obstruction breaks free from the device and flows downstream, it is highly likely that the free material will become trapped in smaller and more tortuous anatomy. In many cases, the physician will no longer be able to use the same retrieval device to again remove the obstruction because the device may be too large and/or immobile to move the device to the site of the new obstruction.

Procedures for treating ischemic stroke by restoring flow within the cerebral vasculature are subject to the above concerns. The brain relies on its arteries and veins to supply oxygenated blood from the heart and lungs and to remove carbon dioxide and cellular waste from brain tissue. Blockages that interfere with this blood supply eventually cause the brain tissue to stop functioning. If the disruption in blood occurs for a sufficient amount of time, the continued lack of nutrients and oxygen causes irreversible cell death. Accordingly, it is desirable to provide immediate medical treatment of an ischemic stroke.

To access the cerebral vasculature, a physician typically advances a catheter from a remote part of the body (typically a leg) through the abdominal vasculature and into the cerebral region of the vasculature. Once within the cerebral vasculature, the physician deploys a device for retrieval of the obstruction causing the blockage. Concerns about dislodged obstructions or the migration of dislodged fragments increases the duration of the procedure at a time when restoration of blood flow is paramount. Furthermore, a physician might be unaware of one or more fragments that dislodge from the initial obstruction and cause blockage of smaller more distal vessels.

Many physicians currently perform thrombectomies (i.e. clot removal) with stents to resolve ischemic stroke. Typically, the physician deploys a stent into the clot in an attempt to push the clot to the side of the vessel and re-establish blood flow. Tissue plasminogen activator ("tPA") is often injected into the bloodstream through an intravenous line to break down a clot. However, it takes time for the tPA to reach the clot because the tPA must travel through the vasculature and only begins to break up the clot once it reaches the clot material. tPA is also often administered to supplement the effectiveness of the stent. Yet, if attempts at clot dissolution are ineffective or incomplete, the physician can attempt to remove the stent while it is expanded against or enmeshed within the clot. In doing so, the physician must effectively drag the clot through the vasculature, in a proximal direction, into a guide catheter located within vessels in the patient's neck (typically the carotid artery). While this procedure has been shown to be effective in the clinic and easy for the physician to perform, there remain some distinct disadvantages to using this approach.

For example, one disadvantage is that the stent may not sufficiently retain the clot as it pulls the clot to the catheter. In such a case, some or all of the clot might remain in the vasculature. Another risk is that, as the stent mobilizes the clot from the original blockage site, the clot might not adhere to the stent as the stent is withdrawn toward the catheter. This is a particular risk when passing through bifurcations and tortuous anatomy. Furthermore, blood flow can carry the clot (or fragments of the clot) into a branching vessel at a bifurcation. If the clot is successfully brought to the end of the guide catheter in the carotid artery, yet another risk is that the clot may be "stripped" or "sheared" from the stent as the stent enters the guide catheter.

In view of the above, there remains a need for improved devices and methods that can remove occlusions from body lumens and/or vessels.

WO2022/058873 discloses an apparatus including one or more longitudinal elements configured to pass through a sheath within a body of a subject, and one or more expandable multilayered electrode elements coupled to the longitudinal elements. The electrode elements are configured to advance to a thrombus in the body while collapsed inside the sheath and to expand distally to the sheath following the advance to the thrombus. US2010/234842 discloses an apparatus including one or more longitudinal elements configured to pass through a sheath within a body of a subject, and one or more expandable multilayered electrode elements coupled to the longitudinal elements. The electrode elements are configured to advance to a thrombus in the body while collapsed inside the sheath and to expand distally to the sheath following the advance to the thrombus. US2016/199127 discloses a tool for ablation of tissue in a living patient comprising: a plurality of ablation electrodes; a basket mounted axially to a shaft, said basket having: a radially contracted configuration wherein supports of said basket are oriented along an axis of said basket for fitting into a channel of a catheter, a distal end of said catheter fitting into a lumen of the living patient; and a radially spread configuration wherein said supports are spread radially away from said axis for holding said plurality of electrodes against an inner wall of said lumen; a cup shaped embolic trap configured to spread to block said lumen to transport of emboli, said embolic trap spreading radially around an apex located along an axis of said basket and distal to said basket; and a manipulation apparatus configured to be accessible from the proximal end of said catheter said manipulation apparatus configured for: reversibly extending and retrieving said shaft including said basket and said plurality of electrodes and said embolic trap through a distal opening of said catheter; and reversibly switching said basket between said radially contracted configuration and said radially spread configuration. WO2021/118868 discloses a medical device for retrieval of material from vessel lumens by electrically enhancing attachment of the material to a medical device. The device includes an elongate core member having a distal portion configured to be intravascularly positioned at a treatment site within a blood vessel lumen, and an interventional element connected to the distal portion of the elongate core member. The interventional element includes a body that is expandable from a first configuration to a second configuration and an electrically conductive radiopaque marker coupled to the body. An electrically conductive lead has a distal portion electrically coupled to the radiopaque marker and a proximal portion configured to be electrically coupled to a current source. US2020/390457 discloses a distal element comprising an expandable mesh for retrieval of material from vessel lumens. A treatment device includes an elongated member having a proximal portion and a distal portion configured to be positioned within a blood vessel at a treatment site at or near a thrombus. A distal element comprising an expandable mesh is coupled to the distal portion of the elongated member via a connection assembly. In an expanded state, at least a portion of the mesh is configured to be in apposition with the blood vessel wall at the treatment site to anchor or stabilize the elongated member with respect to the blood vessel. The distal element can be electrically coupled to an extracorporeal current generator.

### SUMMARY

Mechanical thrombectomy (i.e., clot-grabbing and removal) has been effectively used for treatment of ischemic stroke. Although most clots can be retrieved in a single pass attempt, there are instances in which multiple attempts are needed to fully retrieve the clot and restore blood flow through the vessel. Additionally, there exist complications due to detachment of the clot from the interventional element during the retrieval process as the interventional element and clot traverse through tortuous intracranial vascular anatomy. For example, the detached clot or clot fragments can obstruct other arteries leading to secondary strokes. The failure modes that contribute to clot release during retrieval are: (a) boundary conditions at bifurcations; (b) changes in vessel diameter; and (c) vessel tortuosity, amongst others.

Certain blood components, such as platelets and coagulation proteins, display negative electrical charges. The systems of the present technology provide an interventional element and a signal generator configured to positively charge the interventional element during one or more stages of a thrombectomy procedure. For example, the signal generator may apply a constant or pulsatile direct current (DC) to the interventional element. The positively charged interventional element attracts negatively charged blood components, thereby improving attachment of the thrombus to the interventional element and reducing the number of device passes or attempts necessary to fully retrieve the clot. In some aspects of the present technology, the system includes an interventional element with a proximally positioned electrical engagement portion and a distally positioned mechanical engagement portion. The electrical engagement portion can include a plurality of discrete electrodes (e.g., conductive bands or other suitable structures), and the mechanical engagement portion can include an expandable member such as a self-expandable stent or basket. In operation the electrodes can be disposed within or radially adjacent a thrombus and the expandable member can be disposed at least partially distal to the thrombus. By supplying electrical current to the electrodes and/or the expandable member, the interventional element can engage the thrombus, allowing it to be removed from the blood vessel.

The invention provides a treatment system comprising: a signal generator comprising a first electrical terminal; and a treatment device comprising: an axially elongated electrically conductive core member comprising a proximal end portion configured to be placed in electrical communication with the first electrical terminal and a distal end portion configured to be positioned proximate a thrombus within a lumen of a blood vessel at a treatment site; and an interventional element comprising the distal end portion of the core member, the interventional element further comprising: an electrical engagement portion including a plurality of discrete electrodes spaced apart from one another along the axial direction and configured such that the electrodes are in electrical communication with the conductive core member; and an expandable distal member coupled to the core member and disposed distal to the electrical engagement portion, the expandable distal member configured to expand from a low-profile state into an expanded state into apposition with a wall of the blood vessel at the treatment site when in an unconstrained state, wherein in the expanded state the expandable distal member is configured to engage a thrombus. The electrical engagement portion has a radially outermost dimension that tapers distally such that a radially outermost dimension of a distally located electrode is smaller than a radially outermost dimension of a proximally located electrode, and wherein the distal taper increases flexibility of the electrical engagement portion.

Optionally, the system further comprises a negative electrode in electrical communication with the signal generator, wherein the signal generator, the treatment device, and the negative electrode together form an electrical circuit.

Optionally, the first electrical terminal is positive, the signal generator further comprising a second electrical terminal that is negative.

Optionally, when current is supplied via the signal generator, the interventional element is positively electrically charged to promote adhesion of a thrombus thereto.

Optionally, the system the core member comprises at least one of: a flexible conductive wire and an electrically insulative coating along at least a portion of a length of the flexible conductive wire; or a conductive tube and an electrically insulative coating along at least a portion of a length of the conductive tube.

Optionally, the electrodes comprise portions of the conductive wire or the conductive tube that are uninsulated.

Optionally, the electrodes comprise annular or semi-annular conductive elements extending at least half-way around a circumference of the core member.

Optionally, the electrodes comprise conductive bands disposed over an outer surface of the core member.

Optionally, the bands are separated from one another by electrically insulated regions of the electrical engagement portion, and wherein radially outer surfaces of the bands extend beyond radially outer surfaces of the insulated regions, thereby providing an enlarged surface area of the electrical engagement portion.

Optionally, the expandable distal member comprises a stent or a basket.

Optionally, the expandable distal member comprises a plurality of radiopaque markers coupled to the expandable distal member such that radial distances between the markers is greater when the expandable distal member is in the expanded state than when the expandable distal member is in the low-profile state.

Additional features and advantages of the present technology are described below, and in part will be apparent from the description, or may be learned by practice of the present technology. The advantages of the present technology will be realized and attained by the structure particularly pointed out in the written description and claims hereof as well as the appended drawings.

### BRIEF DESCRIPTION OF THE DRAWINGS

Many aspects of the present technology can be better understood with reference to the following drawings. The components in the drawings are not necessarily to scale. Instead, emphasis is placed on illustrating clearly the principles of the present disclosure.
FIG. 1A shows a perspective view of a system for retrieving material from a body lumen, in accordance with one or more embodiments of the present technology.
FIG. 1B shows an enlarged detail view of a portion of the system shown in FIG. 1A.
FIG. 2 is a side schematic view of an interventional element in accordance with embodiments of the present technology.
FIG. 3 is a side schematic cross-sectional view of an electrical engagement portion of an interventional element in accordance with embodiments of the present technology.
FIG. 4 is a side schematic view of an interventional element in accordance with embodiments of the present technology.
FIGS. 5-7 illustrate side schematic views of a proximal end portion of a treatment device in accordance with embodiments of the present technology.
FIG. 8 illustrates use of a treatment device to remove clot material from a blood vessel lumen in accordance with embodiments of the present technology.

### DETAILED DESCRIPTION.

### I. Select Embodiments of Treatment Systems and Devices

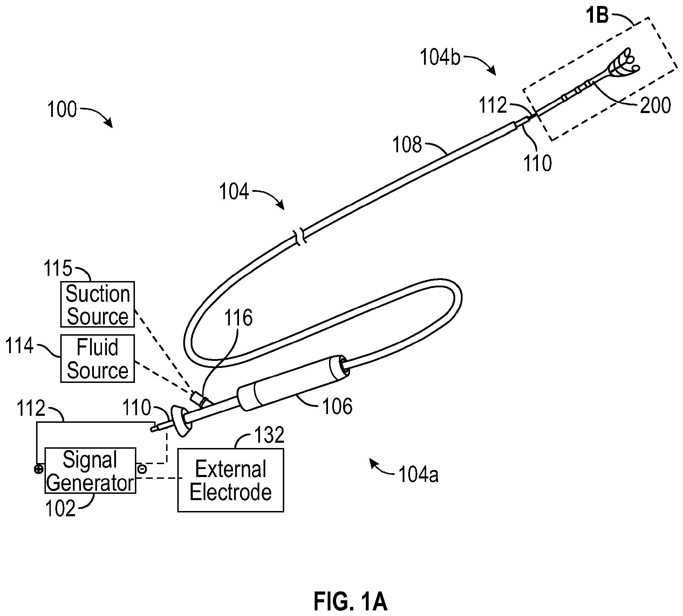

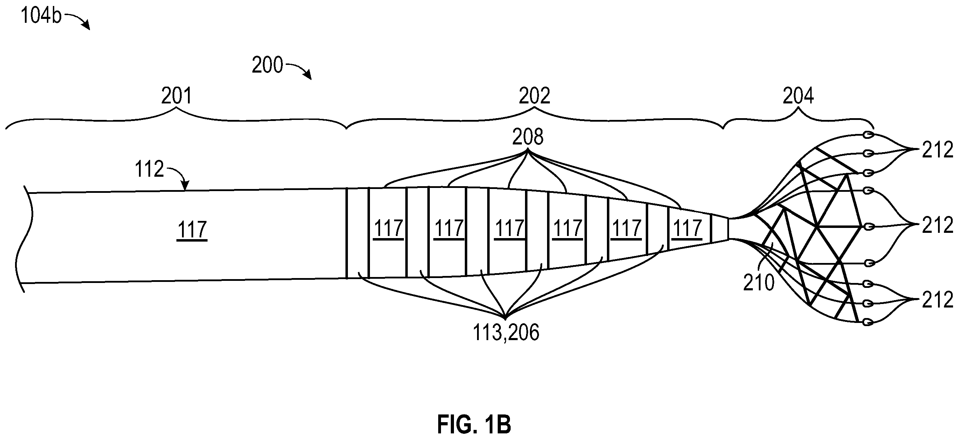

FIG. 1A illustrates a treatment system 100 for removing material from a vessel lumen according to one or more embodiments of the present technology. As shown in FIG. 1A, the system 100 can include a signal generator 102 and a treatment device 104 having a proximal portion 104a coupled to the signal generator 102 and a distal portion 104b configured to be intravascularly positioned within a blood vessel (such as an intracranial blood vessel) at a treatment site at or proximate a thrombus. The treatment device 104 may include an interventional element 200 at the distal portion 104b, a handle 106 at the proximal portion 104a, and a core member 112 extending therebetween. The core member 112 may include an elongate electrically conductive inner body 113 (FIG. 1B), for example a wire, tube (e.g., a metallic hypotube), or other suitable structure. The core member may optionally include electrically insulative material 117 disposed over at least a portion the length of the electrically conductive inner body 113. As described in more detail below, the interventional element 200 can include a proximal electrical engagement portion comprising one or more discrete electrodes and a mechanical engagement portion comprising an expandable member such as a stent, basket, or other suitable structure. In some implementations, the electrical engagement portion comprises a plurality of ring-like electrodes carried by (and/or formed by) the core member 112. In operation, the interventional element 200 can be deployed at or adjacent a thrombus in a blood vessel, and electrical current can be supplied via the signal generator 102. In some examples, the signal generator 102 can positively charge at least a portion of the interventional element 200 (e.g., the electrode(s) of the electrical engagement portion and/or the expandable member of the mechanical engagement portion) to promote adhesion of the thrombus thereto. The interventional element 200 can then be proximally retracted to remove the thrombus from the blood vessel and restore blood flow therethrough.

As shown in FIG. 1A, the system 100 can additionally include a first catheter 108 (such as a guide catheter) and a second catheter 110 (such as a microcatheter) configured to be slidably disposed within a lumen of the first catheter 108, with the core member 112 configured to be slidably disposed within a lumen of the second catheter 110. The core member 112 has a distal portion coupled to the interventional element 200 and is configured to push and pull the interventional element 200 along the vasculature. In some embodiments, the system 100 optionally includes a third catheter, such as a distal access catheter or aspiration catheter (not shown). The third catheter may be configured to be slidably disposed within a lumen of the first catheter 108, and has a lumen configured to receive the second catheter 110 therethrough. As described herein, the signal generator 102 is configured to be coupled to a proximal portion of one or more of the core member 112, the first catheter 108, the second catheter 110, and/or the third catheter to provide an electrically charged environment at the distal portion 104b of the treatment device 104 to promote adhesion of the thrombus to the interventional element 200.

According to some embodiments, the signal generator 102 can include an electrical generator configured to output medically useful electric current. The signal generator 102 can include a power source, one or more electrical terminals for coupling to the treatment device 104, an electrode, or other component, and a suitable controller. The controller can include, for example, a processor coupled to a memory that stores instructions (e.g., in the form of software, code or program instructions executable by the processor or controller) for causing the power source to deliver electric current according to certain parameters provided by the software, code, etc. The power source of the signal generator 102 may include a direct current power supply, an alternating current power supply, and/or a power supply switchable between a direct current and an alternating current. The power source may therefore comprise a battery, transformer, rectifier, a connection to electrical mains or service grid, or any other suitable source of AC or DC electrical power. The signal generator 102 can include a suitable controller that can be used to control various parameters of the energy output by the power source or generator, such as intensity, amplitude, duration, frequency, duty cycle, and polarity. For example, the signal generator 102 can provide a voltage of about 2 volts to about 28 volts and a current of about 0.5 mA to about 20 mA. In some embodiments, the signal generator 102 is configured to deliver current to the interventional element 200 for about 1 minute to about 5 minutes, about 1 minute to about 3 minutes, or at least 2 minutes.

In some embodiments, the signal generator 102 can utilize drive circuitry rather than a processor. For example, the drive circuity can include hardwired circuit elements to provide the desired waveform delivery (or a constant current) rather than a software-based generator. The drive circuitry can include, for example, analog circuit elements (e.g., resistors, diodes, switches, etc.) that are configured to cause the power source to deliver electric current via the first and second terminals according to the desired parameters. For example, the drive circuitry can be configured to cause the power source to deliver periodic waveforms.

With continued reference to FIG. 1A, the first and second catheters (and third catheter when included) can each be formed as a generally tubular member extending along and about a central axis and terminating at a respective distal end. The first catheter 108 may be a balloon-guide catheter having an inflatable balloon or other expandable member (e.g., at or near the distal end thereof) that can be used to anchor the first catheter 108 with respect to a surrounding vessel. In some embodiments, the first catheter 108 does not include an expandable member. The second catheter 110 may be generally constructed to track over a conventional guidewire in the cervical anatomy and into the cerebral vessels associated with the brain. The second catheter 110, for example, can have a length that is at least 125 cm long, and more particularly may be between about 125 cm and about 175 cm long. Other designs and dimensions are contemplated. The third catheter, if included, can be sized and configured to be slidably receive the second catheter 110 therethrough.

According to some embodiments, the bodies of the catheters can be made from various thermoplastics, e.g., polytetrafluoroethylene (PTFE or TEFLON^{®}), fluorinated ethylene propylene (FEP), high-density polyethylene (HDPE), polyether ether ketone (PEEK), etc., which can optionally be lined on the inner surface of the catheters or an adjacent surface with a hydrophilic material such as polyvinylpyrrolidone (PVP) or some other plastic coating. Additionally, either surface can be coated with various combinations of different materials, depending upon the desired results.

As shown in FIG. 1A, the system 100 may additionally include a suction source 115 (e.g., a syringe, a pump, etc.) configured to be fluidly coupled (e.g., via a connector 116) to a proximal portion of one or more of the first catheter 108, the second catheter 110, and/or the third catheter to apply negative pressure therethrough. In these and other embodiments, the system 100 includes a fluid source 114 (e.g., a fluid reservoir, a syringe, pump, etc.) configured to be fluidly coupled (e.g., via the connector 116) to a proximal portion of one or more of the first catheter 108, the second catheter 110, and/or the third catheter to supply fluid (e.g., saline, contrast agents, a drug such as a thrombolytic agent, etc.) to the treatment site.

FIG. 1B is a side schematic view of the distal portion 104b of the treatment device 104. As illustrated, the treatment device 104 includes the core member 112, which includes a proximally positioned pusher portion 201 and a distally positioned electrical engagement portion 202. In some examples, the pusher portion 201 can serve as a structural element for advancing the distal portion 104b of the treatment device 104 through the patient's vasculature, and the pusher portion 201 may be free from electrical or mechanical elements configured to engage a thrombus. A mechanical engagement portion 204 is coupled to the core member 112 and disposed distal to the electrical engagement portion 202. As used herein, the "interventional element 200" can include both the electrical engagement portion 202 of the core member 112, and the mechanical engagement portion 204. Although the illustrated example depicts the electrical engagement portion 202 as part of the core member 112, in some embodiments the electrical engagement portion 202 can be a separate structure that is coupled to a distal end portion of the pusher portion 201. In the illustrated example, the mechanical engagement portion 204 is disposed distal to the electrical engagement portion 202, though in some embodiments the mechanical engagement portion 204 can be disposed within or proximal to the electrical engagement portion 202. The electrical engagement portion 202 can include a plurality of discrete electrodes 206 separated from one another along the longitudinal axis of the treatment device 104 by one or more electrically insulated regions 208.

The core member 112 can include an elongate electrically inner body 113 in electrical communication with the signal generator 102 and the interventional element 200. In various examples, the inner body 113 of the core member 112 can include a wire, tube, coil, combinations thereof, or any other suitable structure. The inner body 113 of the core member 112 can be formed of any suitable electrically conductive material, including stainless steel, nitinol, etc. In some embodiments, the inner body 113 can be covered with an electrically insulative material 117 along at least a portion of its length. This insulative material 117 can include an insulative polymer (e.g., Parylene, PTFE) or other suitable material disposed via dip-coating, heat-shrinking, or any other suitable technique. Certain regions of the core member 112 may be uninsulated, for example a proximal end portion can be uninsulated to facilitate connection to the signal generator, and in some implementations the electrodes 206 of the electrical engagement portion 202 can be defined by regions of the core member 112 in which the insulative material 117 is absent, leaving the inner body 113 exposed on a radially outer surface. As shown in FIG. 1B, the core member 112 can optionally taper in the distal direction such that a radially outermost dimension of the core member 112 decreases distally to a smaller radially outermost dimension at a connection point 213 between the core member 112 and the expandable member 210. In various embodiments, the connection point 213 can take any suitable form, such as a snap-fit engagement, a crimped tubular member, a laser weld, etc.

In the illustrated example, the electrodes 206 take the form of ring-like members or bands that extend circumferentially about the core member 112. The electrodes 206 may provide a plurality of discrete, noncontiguous electrode sections about the central axis. Such sections can be in electrical communication with a common conductive path so as to function collectively as a single electrode, or with multiple separate such paths to allow one or more discrete electrodes to function independently if desired. For example, independent functionality of the electrodes may permit more fine-tuned distribution of electrical current at the treatment site. Additionally or alternatively, if the electrodes are electrically isolated from one another, then one or more of the electrodes may serve as an electrical sensor, which may be used to detect the presence or composition of a blood clot. This may permit the clinician to optimize therapy parameters to account for the clot location and/or properties. The electrodes 206 can include bands, rings, wires, or coils coupled to the core member 112, and/or may be defined by exposed portions of the core member 112 (e.g., regions of the conductive inner body 113 not covered by electrically insulative material 117). In addition to the annular configurations of the electrodes 206 shown in FIGS. 1A and 1B, the electrodes 206 can assume other forms, such as semi-annular electrodes, spiral-shaped electrodes, dot-like electrodes, or any other suitable shape. In some embodiments, the electrodes 206 can extend at least half-way around the circumference of the inner body 113 of the core member 112. In various embodiments, the electrodes 206 can be deposited or "painted" electrodes wherein a conductive coating is coupled to the outer surface of the conductive inner body 113. The electrodes 206 can include platinum, platinum alloys (e.g., 92% platinum and 8% tungsten, 90% platinum and 10% iridium), gold, cobalt-chromium, stainless steel (e.g., 304 or 316), nitinol, and combinations thereof, or any suitable electrically conductive materials, metals or alloys. In some embodiments the electrical engagement portion 202 may include only a single electrode 206 rather than multiple discrete electrodes 206.

The mechanical engagement portion 204 can include one or more structures that are configured to physically engage, interlock with, or otherwise contact and grasp a thrombus to facilitate its removal. In the illustrated example, the mechanical engagement portion 204 includes an expandable member 210 that is coupled to (and optionally in electrical communication with) the electrical engagement portion 202. Instead of or in addition to the expandable member 210, the mechanical engagement portion 204 can include barbs, hooks, protrusions, or other suitable non-expandable structures configured to engage a thrombus. The expandable member 210 can be, for example, a self-expandable stent, basket, mesh, filter, or other suitable structure. In various embodiments, the expandable member 210 can have a low-profile state for delivery through a microcatheter and an expanded state when it is released from the microcatheter or otherwise deployed at the treatment site. In the expanded state, the expandable member 210 can have a radially outermost dimension that is greater than that of the core member 112 and/or the electrical engagement portion 202. For example, the expandable member 210 can have a radially outermost dimension sized and configured to permit the expandable member 210 to come into contact with the vessel wall.

As illustrated in FIG. 1B, the expandable member 210 can include one or more radiopaque markers 212 to facilitate visualization under fluoroscopy. In some implementations, providing a plurality of radiopaque markers 212 allows a clinician to determine visually the expandable member 210 is expanded radially when deployed within a blood vessel. For example, by arranging radiopaque markers along various struts, wires, or other structures within the expandable member 210, the individual radiopaque markers 212 may be radially closer together when the expandable member 210 is in its low-profile, constrained state, and the individual radiopaque markers 212 may be radially separated from one another when the expandable member 210 assumes the expanded state. In some examples, the radiopaque markers 212 can be disposed at a distal end portion of the expandable member 210. The radiopaque markers can include marker bands, coils, or other structures made of radiopaque material such as platinum, platinum-iridium, gold, or other suitable material.

The expandable member 210 can have a low-profile, constrained or compressed configuration (not shown) for intravascular delivery to the treatment site within the second catheter 110, and an expanded configuration for securing and/or engaging clot material and/or for restoring blood flow at the treatment site. The expandable member 210 may self-expand from the low-profile configuration to the expanding configuration upon translation from inside of to outside of the second catheter 110. All or a portion of the expandable member 210 can comprise an electrically conductive material. In some embodiments, the expandable member 210 comprises a laser-cut stent, a braid formed of a plurality of wires (e.g., filaments, threads, sutures, fibers or the like) that have been interwoven to form a structure having openings, or any other suitable structure. The expandable member 210 can be composed of metals, polymers, composites, and/or biologic materials. Polymer materials can include Dacron, polyester, polypropylene, nylon, Teflon, polytetrafluoroethylene (PTFE), tetrafluoroethylene, polyethylene terephthalate, polyactic acid (PLA) silicone, polyurethane, polyethylene, polycarbonate, styrene, polyimide, PEBAX, Hytrel, polyvinyl chloride, high-density polyethylene, low-density polyethylene, polyether ether ketone (PEEK), rubber, latex, and/or other suitable polymers known in the art. Other materials known in the art of elastic implants can also be used. Metal materials can include, but are not limited to, nickel-titanium alloys (e.g. Nitinol), platinum, cobalt-chromium alloys, stainless steel, tungsten or titanium. In certain embodiments, metal filaments may be highly polished and/or surface treated to further improve their hemocompatibility.

In some embodiments, the expandable member 210 may comprise an open cell framework or body and a coupling region extending proximally from the body. In some embodiments, a distal portion of the expandable member 210 can be generally tubular (e.g., cylindrical), and the proximal portion of the expandable member 210 tapers proximally to the coupling region. The proximal taper to the coupling region can permit a small-diameter coupling region to serve as an attachment point for the expandable member 210 even though a distal portion of the expandable member 210 has a significantly larger diameter than the coupling region. Additionally or alternatively, the proximal taper can facilitate resheathing of the expandable member 210, such as by proximally retracting the expandable member 210 into a surrounding catheter. In some embodiments, the expandable member 210 is a mesh structure formed of a superelastic material (e.g., Nitinol) or other resilient or self-expanding material configured to self-expand when released from the delivery catheter. For example, in some embodiments the expandable member 210 may be a stent and/or stentriever, such as Medtronic's SolitaireTM Revascularization Device, Stryker Neurovascular's Trevo^{®} ProVueTM Stentriever, or other suitable devices. In other embodiments, the expandable member 210 may include a plurality of braided filaments. Examples of a suitable expandable member 210 include any of those disclosed in U.S. Patent No. 7,300,458, filed November 5, 2007, U.S. Patent No. 8,940,003, filed November 22, 2010, U.S. Patent No. 9,039,749, filed October 1, 2010, and U.S. Patent No. 8,066,757, filed December 28, 2010.

In some examples, the expandable member 210 can take the form of a basket, filter, or other suitable structure configured to prevent clot material from advancing distally beyond the interventional element 200. In such implementations, the expandable member 210 can be configured to expand into apposition with the vessel wall and define a capture surface configured to prevent the passage of clot material while still allowing some blood flow therethrough.

In some embodiments, some or all of the expandable member 210 is formed by drawn-filled tube ("DFT") wires having a radiopaque core (e.g., platinum, tantalum, gold, tungsten, etc.) surrounded by a superelastic material (e.g., Nitinol, a cobalt-chromium alloy, etc.). The radiopaque core may comprise about 5% to about 50% (e.g., 10%, 15%, 20%, 25%, 30%, 35%, 40%, 45%) of the total-cross-sectional area of the individual wires.

The systems and methods of the present technology can include one or more features of the expandable member 210 to facilitate adhesion of the clot to the expandable member 210. For example, in some embodiments, some or all of the expandable member 210 can be coated with one or more highly conductive materials, such as gold, to improve clot adhesion. In some aspects of the present technology, a working length of the expandable member 210 may be coated with the conductive material while a non-working length of the expandable member 210 may be coated with an insulative material. As used herein, a "working length" can refer to a portion of the expandable member 210 configured to engage with a thrombus, and a "non-working length" can refer to those portions of the expandable member 210 that are not configured to engage with a thrombus (e.g., a proximally tapering region that is not intended to engage with a thrombus). In any case, a portion of the expandable member 210 may be coated with an insulative material. In some instances, insulative coating can improve resistance to corrosion of the expandable member 210. The insulative coating can also serve to focus electrical current to non-insulated regions, thereby achieving higher current density in desired portions which may enable improved clot adhesion.

The core member 112 and the interventional element 200 together may comprise a delivery electrode or conductive path, e.g., for transmitting current from the signal generator 102 to the treatment site. A second electrode or conductive path, e.g., for transmitting current from the treatment site to the signal generator 102, can be provided via any suitable approach, such as utilizing a return electrode carried by a catheter, or one or more external electrodes 132 (FIG. 1A), such as a needle puncturing the patient or a grounding pad applied to the patient's skin. In some embodiments, the negative electrode can be an insulated guide wire having an exposed, electrically conductive portion at its distal end.

In some embodiments, the treatment device 104 can be configured such that, when voltage is applied via the signal generator 102 and the interventional element 200 is placed in the presence of blood (or any other electrolytic medium), current flows from the interventional element 200, through the blood or medium, and to the negative electrode(s). Positive current delivered to the interventional element 200 can attract negatively charged constituents of the clot material, thereby enhancing the grip of the interventional element 200 on the clot material. This enables the interventional element 200 to be used to retrieve the clot material with reduced risk of losing grip on the thrombus or a piece thereof, which can migrate downstream and cause additional vessel blockages in areas of the brain that are more difficult to reach. In some examples, the polarity can be reversed, thereby negatively charging the interventional element 200. This can be useful to, for example, attract positively charged material to the interventional element 200 and/or to break up the clot material.

When current is supplied to the interventional element 200, electrical current will flow through the blood (or other medium) through exposed surfaces of the interventional element 200 that are electrically conductive. As such, current may flow through the electrodes 206 of the electrical engagement portion 202 but may not flow through the insulative regions 208 separating the electrodes 206 from one another. In some examples, the mechanical engagement portion 204 may not be electrically charged, either by providing an insulative coating over the mechanical engagement portion 204 or by coupling the mechanical engagement portion 204 and the electrical engagement portion 202 in such a way that the mechanical engagement portion 204 is not in electrical communication with the electrical engagement portion 202 and/or the core member 112. For instance, the connection point 213 between the core member 112 and the expandable member 210 can be configured such that electrical current traveling along the core member 112 does not pass through the connection point 213 to the expandable member. Additionally or alternatively, the expandable member 210 can be coated with an electrically insulative material along some or all of its length.

In some embodiments, at least a portion of the mechanical engagement portion 204 is electrically charged by the signal generator 102, such that when current is supplied by the signal generator 102, both the electrodes 206 of the electrical engagement portion 202 and the mechanical engagement portion 204 are electrically charged. In the illustrated example, the electrical engagement portion 202 and the mechanical engagement portion 204 may be charged with the same polarity (e.g., both positively charged, thereby promoting attraction of negatively charged blood constituents). In some examples, however, a separate conductive path can be provided between the signal generator 102 and the mechanical engagement portion 204 such that the electrical engagement portion 202 can be coupled to a first terminal of the signal generator 102 and the mechanical engagement portion 204 can be coupled to a second terminal of the signal generator. In such implementations, the separate conductive path can be provided alongside the core member 112 (e.g., as a separate conductive shaft running alongside the core member 112), within a lumen of the core member 112 (e.g., when the core member 112 comprises a hypotube), or any other suitable configuration.

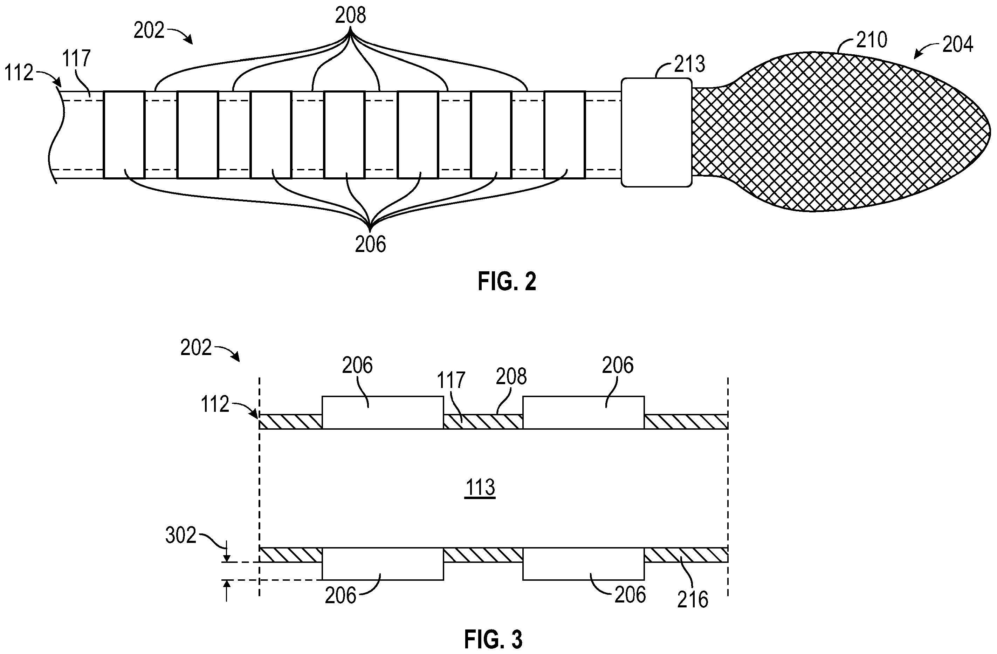

FIG. 2 illustrates a side view of the interventional element 200 in accordance with embodiments of the present technology. As illustrated, the core member 112 includes a conductive inner body 113 that has an insulative material 117 disposed thereover, with the insulative material 117 absent where the electrodes 206 are positioned. The electrodes 206 take the form of bands that extend circumferentially around the core member 112. To form the interventional element 200, the insulative material 117 can first be disposed over the entire outer surface of the inner body 113, and then the particular regions configured to receive the electrodes 206 thereon can have the insulative material 117 removed, for example via laser ablation, manual cutting, or other such approach. In these uninsulated regions, the bands forming the electrodes 206 can be disposed over (e.g., crimped, swaged, etc.) and placed into direct contact with the inner body 113 of the core member 112, thereby placing the electrodes 206 into electrical communication with the inner body 113. As illustrated, the electrodes 206 and the insulative material 117 can each be sized and configured such that the radially outermost surface of the electrical engagement portion 202 is substantially smooth without bumps, protrusions, ridges, or discontinuities in the surface. In the example shown in FIG. 2, the radially outermost dimension of the electrical engagement portion 202 is substantially uniform, although in some examples the radially outermost dimension can vary along its length (e.g., tapering in the distal direction, tapering in the proximal direction, or varying in other ways along the length).

In some embodiments, as shown in FIG. 3, the radially outermost dimension of the electrodes 206 can be greater than the radially outermost dimension of the adjacent insulative regions 208. For example, the electrodes 206 can be formed by crimping or otherwise attaching conductive bands to exposed portions of the core member 112, with the bands being oversized relative to the core member 112 and insulative material 117. As a result, the radially outermost surface of the electrodes 206 can extend beyond the radially outermost surface of the insulative region(s) 208 by a distance 302. In some instances, the resulting ridges, protrusions, bumps, or other such structures can facilitate interlocking with the thrombus when deployed within a blood vessel.

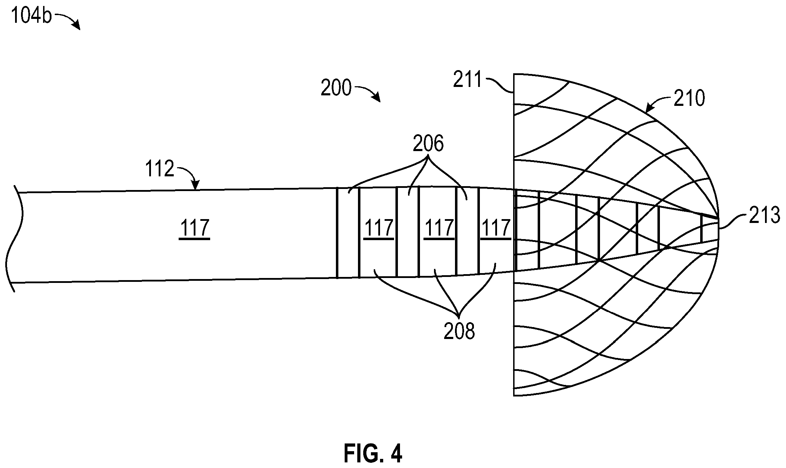

FIG. 4 is a schematic side view of another embodiment of an interventional element 200. In the illustrated example, the interventional element 200 includes a core member 112 carrying and/or defining a plurality of discrete electrodes 206, similar to the examples described above with respect to FIGS. 1B-3. In the example shown in FIG. 4, however, the expandable member 210 takes the form of a basket, funnel, umbrella, or other filter-like structure that at least partially covers a portion of the core member 112. The expandable member 210 can be a mesh formed via laser-cutting, braiding or weaving filaments, or any other suitable technique. As shown in FIG. 4, a distal end 209 of the expandable member 210 can be coupled to the core member 112 via the connection point 213. From the connection point 213, the expandable member 210 extends proximally along the core member 112, with a proximal end 211 of the expandable member 210 defining a proximal opening of the expandable member 210. In various examples, such basket, funnel, umbrella, or other filter-like structures can define an interior region configured to grasp and/or retain clot material therein, particularly when the clot material floats in a distal direction along with blood flow and/or when the expandable member 210 is retracted proximally within the vessel. Although the illustrated example depicts the expandable member 210 extending proximally over some but not all of the electrodes 206, in various embodiments the proximal end 211 of the expandable member can be positioned proximal to the proximalmost electrode 206, distal to the distalmost electrode 206, or any suitable position in between. Moreover, the number of discrete electrodes can vary in this and other configurations, for example there may be 2, 3, 4, 5, 6, 7, 8, 9, 10 or more discrete electrodes 206. In at least some embodiments, there may only be a single electrode 206.

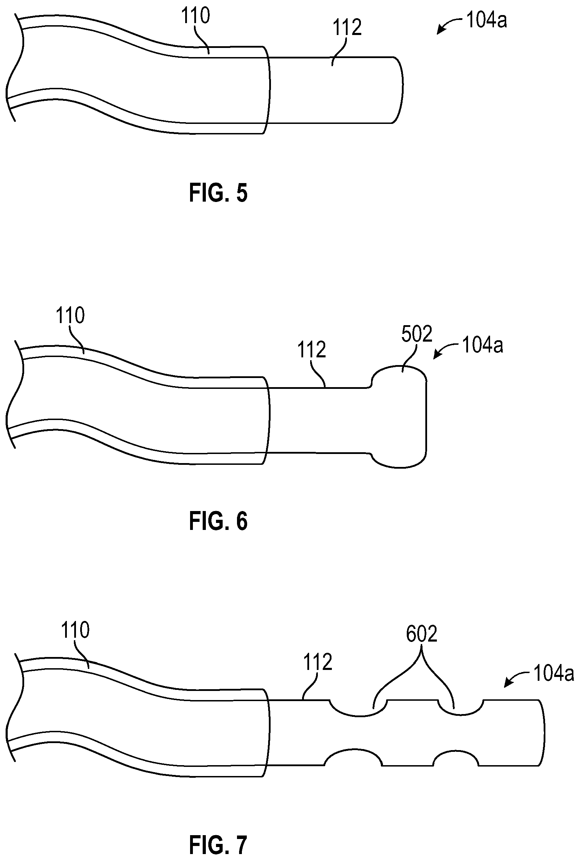

FIGS. 5-7 illustrate various examples of the proximal portion 104a of the treatment device 104. As noted previously, at the proximal portion 104a, the core member 112 can be operably coupled to the signal generator 102 (FIG. 1A). As such, at least a portion of the inner body 113 of the core member 112 may be uncovered by the insulative material 117 such that the core member 112 can be placed in electrical communication with the signal generator 102. This uninsulated portion of the inner body 113 can be achieved by mechanical wire stripping, laser ablation, skiving, cutting, acid etching, or any other suitable technique for removing insulated material from the proximal end of the inner body 113 of the core member 112. In some embodiments, the signal generator 102 can take the form of a handheld current generator, and can have a receptacle configured to receive the proximal portion 104a of the treatment device therein. Examples of such hand-held current generators are described in commonly owned U.S. Application No. 17,304,829, filed June 25, 2021. Depending on the configuration of the signal generator 102, it may be useful to provide engagement features at the proximal portion 104a of the treatment device 104.

In the example shown in FIG. 5, the core member 112 extends proximally beyond a proximal end of the insulative material 117 and retains a generally cylindrical shape. In the example shown in FIG. 6, an engagement feature 502 is disposed at a proximal end of the core member 112. The engagement feature 502 can be an expanded-diameter region of the core member 112, a ball, ridge, bump, or other radially outwardly extending protrusion configured to facilitate engagement with the signal generator 102. In the example shown in FIG. 7, in contrast, the engagement features 602 take the form of recesses, grooves, detents, or other forms in which a radially outermost dimension of the core member 112 is reduced. These recesses or other such structures can extend partially or completely circumferentially around the core member 112. Such reduced-diameter regions can likewise facilitate engagement with a signal generator 102, for example when a signal generator 102 includes prongs, barbs, or other structures that engage the core member 112 by pressing radially inwardly to interlock with the engagement features 602.

### II. Select Methods of Use

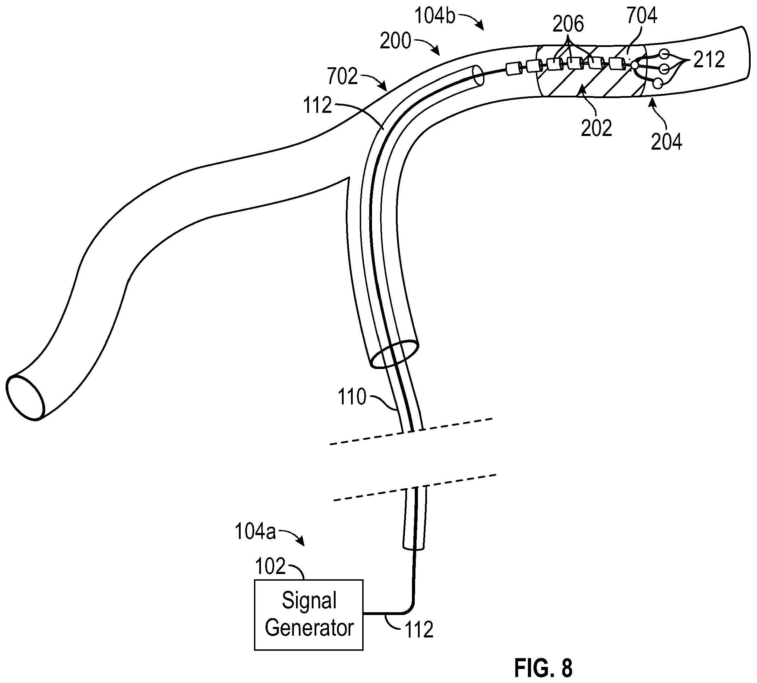

FIG. 8 illustrates an example method of using the treatment device 104 to remove clot material from a blood vessel. In use, the second catheter 110 (e.g., a microcatheter) may be advanced so that a distal portion of the second catheter 110 is positioned at or near the clot material CM. In some embodiments, the second catheter 110 may be positioned such that a distal terminus of the second catheter 110 is distal of the clot material CM. The interventional element 200 may then be advanced through the second catheter 110 in a low-profile configuration until a distal terminus of the interventional element 200 is at or adjacent the distal terminus of the second catheter 110. In particular, a distal terminus of the expandable member 210 can be disposed distal to a distal end of the clot material CM.

In the configuration shown in FIG. 8, the second catheter 110 has been withdrawn proximally relative to the interventional element 200 to release the interventional element 200, thereby allowing the expandable member 210 of the interventional element 200 to self-expand within and/or distal to the clot material CM. As shown, the radiopaque markers 212 can indicate the radial expansion of the expandable member 210, since the markers 212 will be closer together in an unexpanded state and further apart in an expanded state. This may indicate that the distal end portion of the expandable member 210 is distal to the thrombus (i.e., the thrombus is not compressing the expandable member 210 and preventing its full expansion), which may indicate a desired positioning of the interventional element 200. As illustrated, once the interventional element 200 is deployed, the electrodes 206 of the electrical engagement portion 202 are at least partially disposed within or radially adjacent to the clot material CM. In some embodiments, the expandable member 210 is configured to expand into contact with the wall of the vessel V, or the interventional element 200 may expand to a diameter that is less than that of the blood vessel lumen such that the interventional element 200 does not engage the entire circumference of the blood vessel wall.

Once the interventional element 200 has been released from the catheter 110, and the expandable member 210 is expanded, the expandable member 210 may grip the at least a portion of the clot material CM by virtue of its ability to mechanically interlock with the clot material CM. The signal generator 102, which is electrically coupled to the proximal end of the core member 112, can deliver a current to the interventional element 200 before or after the interventional element 200 has been released from the second catheter 110 into the blood vessel and/or expanded into the clot material CM. The electrical current can promote adhesion of the thrombus to the electrodes 206 of the electrical engagement portion 202 and also to the expandable member 210 (in implementations in which the expandable member 210 is in electrical communication with the signal generator 102). The interventional element 200 can be left in place or manipulated within the vessel V for a desired time period while the electrical signal is being delivered. Positive current delivered to the interventional element 200 can attract negatively charged constituents of the clot material CM to positively charged portions of the interventional element (e.g., the electrodes 206 and/or the expandable member 210), thereby enhancing the grip of the interventional element 200 on the clot material CM. This allows the interventional element 200 to be used to retrieve the clot material CM with reduced risk of losing grip on the thrombus or a piece thereof, which can migrate downstream and cause additional vessel blockages in areas of the brain that are more difficult to reach.

While the interventional element 200 is engaged with the clot material CM, the core member 112 can be pulled proximally until the interventional element 200 with the engaged clot material CM is positioned within a surrounding catheter (e.g., the first and/or third catheter). The catheters, interventional element 200, and associated clot material CM may then be withdrawn from the patient. During this retraction, the interventional element 200 can grip the clot material CM electrically and/or electrostatically. (As used herein with reference to gripping or retrieving thrombus or other vascular/luminal material, or to apparatus for this purpose, "electrical" and its derivatives will be understood to include "electrostatic" and its derivatives.) Accordingly, the interventional element 200 can maintain an enhanced or electrically and/or electrostatically enhanced grip on the clot material CM during retraction. In other embodiments, the signal generator 102 may cease delivery of electrical signals to the interventional element 200 prior to retraction of the interventional element 200 with respect to the vessel V. In some embodiments, the interventional element 200 and clot material CM form a removable, integrated thrombus-device mass wherein the connection of the thrombus to the device is electrically enhanced, e.g., via the application of current as discussed herein.

One or more optional processes may be performed before, during, and/or after deployment of the interventional element 200. For example, in some methods of the present technology, a guidewire (not shown) may be advanced to the treatment site and pushed through the clot material CM until a distal portion of the guidewire is distal of the clot material CM. The guidewire may be advanced through one or more of the first, second, and/or third catheters, and one or more of the catheters may be advanced over the guidewire. The guidewire may be insulated along at least a portion of its length (e.g., with parylene, PTFE, etc.), with exposed portions permitting electrical communication with the signal generator 102 and the interventional element 200. For example, in some embodiments a distal portion of the guidewire may be exposed and the guidewire may be positioned at the treatment site such that the exposed portion of the guidewire is distal of the clot material CM. A proximal end of the guidewire may be coupled to the signal generator such that the exposed portion of the guidewire functions as a return electrode. In some embodiments, the guidewire may be coupled to the positive terminal of the power source and the exposed portion functions as a delivery electrode. The guidewire may be used as a delivery or return electrode with any delivery or return electrode carried by any component of the system (e.g., one or more of the first, second, and/or third catheters, the interventional element 200, etc.).

In some methods a fluid may be delivered to the treatment site via the second and/or third catheter while current is being delivered to the interventional element 200. Fluid delivery may occur before or while the interventional element 200 is engaging the thrombus, and may coincide with the entire duration of current delivery or just a portion thereof. Additionally or alternatively, it may be especially beneficial to arrest flow (e.g., via a flow arrest element on the first or second catheter 108, 110) while the interventional element 200 is charged, and while expanding the interventional element 200 within the thrombus and/or when withdrawing the thrombus proximally.

Although the presence of blood flow at the treatment site is believed to reduce adhesion between an electrically charged interventional element and a blood clot, infusion of a fluid having a higher ion concentration than blood can increase the electrical conductivity at the treatment site, thereby providing an improved environment for electrically enhanced clot adhesion as compared to the presence of blood alone. In some embodiments, infusion of the fluid may occur in the presence of blood flow, or without blood flow present (the latter condition being induced, for example, by inflation of an expandable flow-arrest element on a catheter). Suitable fluids include, for example, saline, contrast solution, and other fluids having a higher ion concentration than blood. Additionally, the delivery of fluid at the treatment site may also reduce new clot formation on the interventional element 200, which may occur in the presence of blood and direct or pulsatile electric current.

In some instances aspiration may be applied to the treatment site via an aspiration catheter. For example, following deployment of the interventional element 200, the second catheter 110 can be retracted and removed from the lumen of a third catheter. The treatment site can then be aspirated via the third catheter, for example via a suction source (such as suction source 115) coupled to a proximal portion of the third catheter. In some embodiments, following expansion of the interventional element 200, the treatment site is aspirated concurrently with supplying electrical energy to the interventional element 200 via the signal generator 102. By combining aspiration with the application of electrical energy, any newly formed clots (e.g., any clots formed that are attributable at least in part to the application of electrical energy), or any clot pieces that are broken loose during the procedure, can be pulled into the third catheter, thereby preventing any such clots from being released downstream of the treatment site. As a result, concurrent aspiration may permit the use of higher power or current levels delivered to the interventional element 200 without risking deleterious effects of new clot formation. Additionally, aspiration can capture any gas bubbles formed along the interventional element 200 or other component during application of electrical energy to the interventional element 200, which can improve patient safety during the procedure.

### III. Select Waveforms for Electrically Enhanced Retrieval

Although the waveforms and other power delivery parameters disclosed herein can be used with the devices and methods described above with respect to FIGS. 1A-8, the waveforms and other parameters are also applicable to other device configurations and techniques. For example, the return electrode can be provided along the catheter wall, as a separate conductive member extending within the catheter lumen, as a needle electrode provided elsewhere in the body, etc. In each of these device configurations, the power delivery parameters and waveforms can be beneficially employed to promote clot adhesion without damaging surrounding tissue. Additionally, although the waveforms and other power delivery parameters disclosed herein may be used for treating a cerebral or intracranial embolism, other applications and other embodiments in addition to those described herein are within the scope of the technology. For example, the waveforms and power delivery parameters disclosed herein may be used to electrically enhance removal of emboli from body lumens other than blood vessels (e.g., the digestive tract, etc.) and/or may be used to electrically enhance removal of emboli from blood vessels outside of the brain (e.g., pulmonary blood vessels, blood vessels within the legs, etc.).

While applying a continuous uniform direct current (DC) electrical signal to positively charge the interventional element and/or catheter can improve attachment to the thrombus, this can risk damage to surrounding tissue (e.g., ablation), and sustained current at a relatively high level may also be thrombogenic (i.e., may generate new clots). For achieving effective clot-grabbing without ablating tissue or generating substantial new clots at the treatment site, periodic waveforms have been found to be particularly useful. Without being bound by theory, the clot-adhesion effect appears to be most closely related to the peak current of the delivered electrical signal. Periodic waveforms can advantageously provide the desired peak current without delivering excessive total energy or total electrical charge. Periodic, non-square waveforms in particular are well suited to deliver a desired peak current while reducing the amount of overall delivered energy or charge as compared to either uniform applied current or square waveforms.

Periodic waveforms that can be used with the devices and methods described above with respect to FIGS. 1A-8, including as pulsed direct current. In various embodiments, the signal comprises a continuous uniform DC electrical signal, a time-varying monopolar signal (e.g., a square wave, triangular wave, etc. with a single polarity), an AC signal, or any combination thereof.

The waveform shape (e.g., pulse width, duty cycle, amplitude) and length of time can each be selected to achieve desired power delivery parameters, such as overall electrical charge, total energy, and peak current delivered to the interventional element and/or catheter. In some embodiments, the overall electrical charge delivered to the interventional element and/or catheter can be between about 30-1200 mC, or between about 120-600 mC. According to some embodiments, the total electrical charge delivered to the interventional element and/or catheter may be less than 600 mC, less than 500 mC, less than 400 mC, less than 300 mC, less than 200 mC, or less than 100 mC.

In some embodiments, the total energy delivered to the interventional element and/or aspiration catheter can be between about 0.75-24,000 mJ, or between about 120-24,000 mJ, or between about 120-5000 mJ. According to some embodiments, the total energy delivered to the interventional element and/or aspiration catheter may be less than 24,000 mJ, less than 20,000 mJ, less than 15,000 mJ, less than 10,000 mJ, less than 5,000 mJ, less than 4,000 mJ, less than 3,000 mJ, less than 2000 mJ, less than 1,000 mJ, less than 900 mJ, less than 800 mJ, less than 700 mJ, less than 600 mJ, less than 500 mJ, less than 400 mJ, less than 300 mJ, or less than 200 mJ, or less than 120 mJ, or less than 60 mJ, or less than 48 mJ, or less than 30 mJ, or less than 12 mJ, or less than 6 mJ, or less than 1.5 mJ.

In some embodiments, the peak current delivered can be between about 0.5-20 mA, or between about 0.5-5 mA. According to some embodiments, the peak current delivered may be greater than 0.5 mA, greater than 1 mA, greater than 1.5 mA, greater than 2 mA, greater than 2.5 mA, or greater than 3 mA.

The duration of power delivery is another important parameter that can be controlled to achieve the desired clot-adhesion effects without damaging tissue at the treatment site or generating new clots. In at least some embodiments, the total energy delivery time can be no more than 1 minute, no more than 2 minutes, no more than 3 minutes, no more than 4 minutes, no more than 5 minutes, or between 1 and 5 minutes. According to some embodiments, the total energy delivery time may be less about 30 seconds, less than about 1 minute, less than about 90 seconds, or less than about 2 minutes. As used herein, the "total energy delivery time" refers to the time period during which the waveform is supplied to the interventional element and/or catheter (including those periods of time between pulses of current).

The duty cycle of the applied electrical signal can also be selected to achieve the desired clot-adhesion characteristics without ablating tissue or promoting new clot formation. In some embodiments, the duty cycle can be between about 5% about 99% or between about 5% to about 20%. According to some embodiments, the duty cycle may be about 10%, about 20%, about 30%, about 40%, or about 50%. In yet other embodiments, a constant current may be used, in which the duty cycle is 100%. For 100% duty cycle embodiments, a lower time or current may be used to avoid delivering excess total energy to the treatment site.

### IV. Conclusion

Although many of the embodiments are described herein with respect to devices, systems, and methods for treating a cerebral or intracranial embolism, other applications and other embodiments in addition to those described herein are within the scope of the technology. For example, the systems and methods of the present technology may be used to remove emboli from body lumens other than blood vessels (e.g., the digestive tract, etc.) and/or may be used to remove emboli from blood vessels outside of the brain (e.g., pulmonary, abdominal, cervical, or thoracic blood vessels, or peripheral blood vessels including those within the legs or arms, etc.). In addition, the systems and methods of the present technology may be used to remove luminal obstructions other than clot material (e.g., plaque, resected tissue, foreign material, kidney stones, etc.).

This disclosure is not intended to be exhaustive or to limit the present technology to the precise forms disclosed herein. Although specific embodiments are disclosed herein for illustrative purposes, various equivalent modifications are possible without deviating from the present technology, as those of ordinary skill in the relevant art will recognize. In some cases, well-known structures and functions have not been shown and/or described in detail to avoid unnecessarily obscuring the description of the embodiments of the present technology. Although steps of methods may be presented herein in a particular order, in alternative embodiments the steps may have another suitable order. Similarly, certain aspects of the present technology disclosed in the context of particular embodiments can be combined or eliminated in other embodiments. Furthermore, while advantages associated with certain embodiments may have been disclosed in the context of those embodiments, other embodiments can also exhibit such advantages, and not all embodiments need necessarily exhibit such advantages or other advantages disclosed herein to fall within the scope of the present technology. Accordingly, this disclosure and associated technology can encompass other embodiments not expressly shown and/or described herein. The invention is defined in the appended claims.

Throughout this disclosure, the singular terms "a," "an," and "the" include plural referents unless the context clearly indicates otherwise. Similarly, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. Additionally, the terms "comprising" and the like are used throughout this disclosure to mean including at least the recited feature(s) such that any greater number of the same feature(s) and/or one or more additional types of features are not precluded. Directional terms, such as "upper," "lower," "front," "back," "vertical," and "horizontal," may be used herein to express and clarify the relationship between various elements. It should be understood that such terms do not denote absolute orientation. Reference herein to "one embodiment," "an embodiment," or similar formulations means that a particular feature, structure, operation, or characteristic described in connection with the embodiment can be included in at least one embodiment of the present technology. Thus, the appearances of such phrases or formulations herein are not necessarily all referring to the same embodiment. Furthermore, various particular features, structures, operations, or characteristics may be combined in any suitable manner in one or more embodiments.

## Claims

1. A treatment system (100) comprising:
a signal generator (102) comprising a first electrical terminal; and
a treatment device (100) comprising:
an axially elongated electrically conductive core member (112) comprising a proximal end portion (104a) configured to be placed in electrical communication with the first electrical terminal and a distal end portion (104b) configured to be positioned proximate a thrombus within a lumen of a blood vessel at a treatment site; and
an interventional element (200) comprising the distal end portion (104b) of the core member (112), the interventional element further comprising:
an electrical engagement portion (202) including a plurality of discrete electrodes (206) spaced apart from one another along a longitudinal axial direction and configured such that the discrete electrodes (206) are in electrical communication with the core member (112); and
an expandable distal member (104b) coupled to the core member (112) and disposed distal to the electrical engagement portion (202), the expandable distal member (104b) configured to expand from a low-profile state into an expanded state into apposition with a wall of the blood vessel at the treatment site when in an unconstrained state, wherein in the expanded state the expandable distal member (104b) is configured to engage the thrombus, wherein the electrical engagement portion (202) has a radially outermost dimension that tapers distally such that a radially outermost dimension of a most distally located electrode (206) is smaller than a radially outermost dimension of a most proximally located electrode (206),
and wherein the distal taper increases flexibility of the electrical engagement portion (202).

2. The system (100) of claim 1, further comprising a negative electrode in electrical communication with the signal generator (102), wherein the signal generator (102), the treatment device (100), and the negative electrode together form an electrical circuit.

3. The system (100) of claim 1 or claim 2, wherein the first electrical terminal is positive, the signal generator (102) further comprising a second electrical terminal that is negative.

4. The system (100) of any preceding claim, wherein, when current is supplied via the signal generator (102), the interventional element is positively electrically charged to promote adhesion of the thrombus thereto.

5. The system (100) of any preceding claim, wherein the core member (112) comprises at least one of:
a flexible conductive wire and an electrically insulative coating along at least a portion of a length of the flexible conductive wire; or
a conductive tube and an electrically insulative coating along at least a portion of a length of the conductive tube.

6. The system (100) of claim 5, wherein the discrete electrodes (206) comprise portions of the conductive wire or the conductive tube that are uninsulated.

7. The system (100) of any preceding claim, wherein the discrete electrodes (206) comprise annular or semi-annular conductive elements extending at least half-way around a circumference of the core member (112).

8. The system (100) of any of claims 1 to 7, wherein the discrete electrodes (206) comprise conductive bands disposed over an outer surface of the core member (112).

9. The system (100) of claim 9, wherein the bands are separated from one another by electrically insulated regions of the electrical engagement portion (202), and wherein radially outer surfaces of the bands extend beyond radially outer surfaces of the insulated regions, thereby providing an enlarged surface area of the electrical engagement portion (202).

10. The system (100) of any preceding claim, wherein the expandable distal member (104b) comprises a stent or a basket.

11. The system (100) of any preceding claim, wherein the expandable distal member (104b) comprises a plurality of radiopaque markers (212) coupled to the expandable distal member (104b) such that radial distances between the markers (212) are greater when the expandable distal member (104b) is in the expanded state than when the expandable distal member (104b) is in the low-profile state.

## Patentansprüche

1. Behandlungssystem (100), umfassend:
einen Signalgenerator (102), umfassend einen ersten elektrischen Anschluss; und
eine Behandlungsvorrichtung (100), umfassend:
ein axial verlängertes elektrisch leitfähiges Kernelement (112), umfassend einen proximalen Endabschnitt (104a), der konfiguriert ist, um in elektrischer Verbindung mit dem ersten elektrischen Anschluss zu stehen, und einen distalen Endabschnitt (104b), der konfiguriert ist, um in der Nähe eines Thrombus innerhalb eines Lumens eines Blutgefäßes an einer Behandlungsstelle positioniert zu werden; und
ein interventionelles Element (200), umfassend den distalen Endabschnitt (104b) des Kernelements (112), das interventionelle Element ferner umfassend:
einen elektrischen Eingriffsabschnitt (202), der eine Vielzahl diskreter Elektroden (206) umfasst, die entlang einer Längsaxialrichtung voneinander beabstandet sind und derart konfiguriert sind, dass die diskreten Elektroden (206) in elektrischer Verbindung mit dem Kernelement (112) stehen; und
ein ausdehnbares distales Element (104b), das mit dem Kernelement (112) gekoppelt und distal zu dem elektrischen Eingriffsabschnitt (202) angeordnet ist, wobei das ausdehnbare distale Element (104b) konfiguriert ist, um sich von einem Zustand mit geringem Profil in einen ausgedehnten Zustand in Apposition mit einer Wand des Blutgefäßes an der Behandlungsstelle auszudehnen, wenn es in einem uneingeschränkten Zustand ist, wobei das ausdehnbare distale Element (104b) in dem ausgedehnten Zustand konfiguriert ist, um den Thrombus in Eingriff zu nehmen, wobei der elektrische Eingriffsabschnitt (202) eine radial äußerste Abmessung aufweist, die sich derart distal verjüngt, dass eine radial äußerste Abmessung einer am weitesten distal gelegenen Elektrode (206) kleiner als eine radial äußerste Abmessung einer am weitesten proximal gelegenen Elektrode (206) ist, und wobei die distale Verjüngung eine Flexibilität des elektrischen Eingriffsabschnitts (202) erhöht.

2. System (100) nach Anspruch 1, ferner umfassend eine negative Elektrode in elektrischer Verbindung mit dem Signalgenerator (102), wobei der Signalgenerator (102), die Behandlungsvorrichtung (100) und die negative Elektrode zusammen einen Stromkreis ausbilden.

3. System (100) nach Anspruch 1 oder 2, wobei der erste elektrische Anschluss positiv ist, der Signalgenerator (102) ferner umfassend einen zweiten elektrischen Anschluss, der negativ ist.

4. System (100) nach einem der vorstehenden Ansprüche, wobei, wenn Strom über den Signalgenerator (102) zugeführt wird, das interventionelle Element positiv elektrisch geladen wird, um eine Haftung des Thrombus daran zu fördern.

5. System (100) nach einem der vorstehenden Ansprüche, wobei das Kernelement (112) mindestens eines umfasst von:
einen flexiblen leitfähigen Draht und eine elektrisch isolierende Beschichtung entlang mindestens eines Teils einer Länge des flexiblen leitfähigen Drahts; oder
ein leitfähiges Rohr und eine elektrisch isolierende Beschichtung entlang mindestens eines Abschnitts der Länge des leitfähigen Rohrs.

6. System (100) nach Anspruch 5, wobei die diskreten Elektroden (206) Abschnitte des leitfähigen Drahts oder des leitfähigen Rohrs umfassen, die nicht isoliert sind.

7. System (100) nach einem der vorstehenden Ansprüche, wobei die diskreten Elektroden (206) ringförmige oder halbringförmige leitfähige Elemente umfassen, die sich mindestens halbwegs um einen Umfang des Kernelements (112) erstrecken.

8. System (100) nach einem der Ansprüche 1 bis 7, wobei die diskreten Elektroden (206) leitfähige Bänder umfassen, die über einer äußeren Oberfläche des Kernelements (112) angeordnet sind.

9. System (100) nach Anspruch 9, wobei die Bänder durch elektrisch isolierte Bereiche des elektrischen Eingriffsabschnitts (202) voneinander getrennt sind und wobei sich radial äußere Oberflächen der Bänder über radial äußere Oberflächen der isolierten Bereiche hinaus erstrecken, wodurch ein vergrößerter Oberflächeninhalt des elektrischen Eingriffsabschnitts (202) bereitgestellt wird.

10. System (100) nach einem der vorstehenden Ansprüche, wobei das ausdehnbare distale Element (104b) einen Stent oder einen Korb umfasst.

11. System (100) nach einem der vorstehenden Ansprüche, wobei das ausdehnbare distale Element (104b) eine Vielzahl von röntgenstrahlenundurchlässigen Markern (212) umfasst, die mit dem ausdehnbaren distalen Element (104b) derart gekoppelt sind, dass die radialen Abstände zwischen den Markern (212) größer sind, wenn das ausdehnbare distale Element (104b) in dem ausgedehnten Zustand ist, als wenn das ausdehnbare distale Element (104b) in dem Zustand mit geringem Profil ist.

## Revendications

1. Système de traitement (100) comprenant :
un générateur de signaux (102) comprenant une première borne électrique ; et
un dispositif de traitement (100), comprenant :
un élément central électriquement conducteur allongé axialement (112) comprenant une partie d'extrémité proximale (104a) conçue pour être placée en communication électrique avec la première borne électrique et une partie d'extrémité distale (104b) conçue pour être positionnée à proximité d'un thrombus à l'intérieur d'une lumière d'un vaisseau sanguin au niveau d'un site de traitement ; et
un élément interventionnel (200) comprenant la partie d'extrémité distale (104b) de l'élément central (112), l'élément interventionnel comprenant en outre :
une partie de mise en prise électrique (202) comportant une pluralité d'électrodes distinctes (206) espacées les unes des autres le long d'une direction axiale longitudinale et configurées de telle sorte que les électrodes distinctes (206) soient en communication électrique avec l'élément central (112) ; et
un élément distal expansible (104b) accouplé à l'élément central (112) et disposé distalement par rapport à la partie de mise en prise électrique (202), l'élément distal expansible (104b) étant conçu pour s'expanser d'un état de profil bas à un état expansé en apposition avec une paroi du vaisseau sanguin au niveau du site de traitement lorsqu'il est dans un état non contraint, dans lequel à l'état expansé l'élément distal expansible (104b) est conçu pour venir en prise avec le thrombus, dans lequel la partie de mise en prise électrique (202) a une dimension radialement la plus extérieure qui s'amincit distalement de telle sorte qu'une dimension radialement la plus extérieure d'une électrode située le plus distalement (206) est plus petite qu'une dimension radialement la plus extérieure d'une électrode située le plus proximalement (206), et dans lequel l'amincissement distal augmente la flexibilité de la partie de mise en prise électrique (202).

2. Système (100) selon la revendication 1, comprenant en outre une électrode négative en communication électrique avec le générateur de signaux (102), dans lequel le générateur de signaux (102), le dispositif de traitement (100), et l'électrode négative, forment ensemble un circuit électrique.

3. Système (100) selon la revendication 1 ou la revendication 2, dans lequel la première borne électrique est positive, le générateur de signaux (102) comprenant en outre une seconde borne électrique qui est négative.

4. Système (100) selon l'une quelconque revendication précédente, dans lequel, lorsque le courant est fourni par le biais du générateur de signaux (102), l'élément interventionnel est chargé électriquement de manière positive pour favoriser l'adhésion du thrombus à celui-ci.

5. Système (100) selon l'une quelconque revendication précédente, dans lequel l'élément central (112) comprend au moins l'un parmi :
un fil conducteur flexible et un revêtement électriquement isolant le long d'au moins une partie d'une longueur du fil conducteur flexible ; ou
un tube conducteur et un revêtement électriquement isolant le long d'au moins une partie d'une longueur du tube conducteur.

6. Système (100) selon la revendication 5, dans lequel les électrodes distinctes (206) comprennent des parties du fil conducteur ou du tube conducteur qui ne sont pas isolées.

7. Système (100) selon l'une quelconque revendication précédente, dans lequel les électrodes distinctes (206) comprennent des éléments conducteurs annulaires ou semi-annulaires s'étendant au moins sur la moitié d'une circonférence de l'élément central (112).

8. Système (100) selon l'une quelconque des revendications 1 à 7, dans lequel les électrodes distinctes (206) comprennent des bandes conductrices disposées sur une surface extérieure de l'élément central (112).

9. Système (100) selon la revendication 9, dans lequel les bandes sont séparées les unes des autres par des régions électriquement isolées de la partie de mise en prise électrique (202), et dans lequel les surfaces radialement extérieures des bandes s'étendent au-delà des surfaces radialement extérieures des régions isolées, fournissant ainsi une surface élargie de la partie de mise en prise électrique (202).

10. Système (100) selon l'une quelconque revendication précédente, dans lequel l'élément distal expansible (104b) comprend une endoprothèse ou un panier.

11. Système (100) selon l'une quelconque revendication précédente, dans lequel l'élément distal expansible (104b) comprend une pluralité de marqueurs radio-opaques (212) accouplés à l'élément distal expansible (104b) de telle sorte que les distances radiales entre les marqueurs (212) sont plus grandes lorsque l'élément distal extensible (104b) est dans l'état expansé que lorsque l'élément distal expansible (104b) est dans l'état de profil bas.
